# EUROPEAN PATENT SPECIFICATION

(11) **EP 2 849 106 B1**
(45) Date of publication and mention of the grant of the patent: **12.06.2019**
(21) Application number: 14184927.3
(22) Date of filing: 16.09.2014
(51) Int. Cl.: G06F 16/58

(54) **Medical image managing method and apparatus**
Verfahren und Vorrichtung zur Verwaltung medizinischer Bilder
Procédé de gestion d'image médicale et appareil

(30) Priority: 17.09.2013 KR 20130112068
(43) Date of publication of application: 18.03.2015
(73) Proprietor: Samsung Electronics Co., Ltd., Gyeonggi-do 443-742 (KR)
(72) Inventor: Dutta, Rakesh, Gyeonggi-do (KR)
(74) Representative: Grootscholten, Johannes A.M.

(56) References cited:
- EP-A2- 1 176 538
- WO-A2-2006/040258
- DE-A1-102007 040 838
- US-A1- 2008 212 855

## Description

### BACKGROUND

### 1. Field

One or more exemplary embodiments consistent with the present disclosure relate to a medical image managing method and apparatus, and more particularly, to a method and an apparatus that manage a medical image by transmitting, deleting, and recovering the medical image.

### 2. Description of the Related Art

A medical image is captured by a medical photographing apparatus such as a magnetic resonance imaging (MRI) apparatus, a computed tomography (CT) apparatus, a position emission tomography (PET) apparatus, or an X-ray apparatus, and may be stored in a storage unit of a central control apparatus. A user may check the medical image stored in the storage unit of the central control apparatus, and use the medical image for a diagnosis of a patient. However, the storage unit of the central control apparatus has a limited storage capacity, and thus, cannot store all medical images captured by the medical image photographing apparatus. In the related art, in order to overcome the limited storage capacity of the storage unit, a user makes backup copies of the medical images stored in the storage unit to respectively store the backup copies in a plurality of external storage devices. However, when the user intends to check a desired medical image deleted from the storage unit of the central control apparatus, the user may not know which of the external storage devices has been used to store a backup copy of the desired medical image, and due to this uncertainty, diagnosis efficiency of a patient is reduced. Corresponding methods are exemplary disclosed in the prior art documents EP 1 176 538 A2, WO 2006/040258 A2 and DE 10 2007 040838.

Moreover, in the related art, since a user directly performs an operation of deleting and recovering a medical image, a problem occurs in which the medical image may be permanently deleted by the user and thus cannot be recovered.

Therefore, it is required to develop a method that reduces mistakes in an operation of deleting and recovering a medical image, thereby enabling the medical image to be easily recovered.

### SUMMARY

One or more exemplary embodiments include a medical image managing method and apparatus that may easily recover a medical image deleted from the medical image managing apparatus.

Additional aspects will be set forth in part in the description which follows and, in part, will be apparent from the description, or may be learned by practice of the exemplary embodiments.

According to one or more exemplary embodiments, provided is a method that manages a medical image by deleting and recovering the medical image.

According to one or more exemplary embodiments, there is provided a medical image managing method to be performed using a medical image managing apparatus, the medical image managing method including: transmitting a backup copy of a medical image, which medical image is stored in the medical image managing apparatus, to a first external storage device which is selected from among a plurality of external storage devices; storing mapping information comprising information about the medical image and information indicating the first external storage from among a plurality of external storage devices, to which the backup copy of the medical image is transmitted; setting a deleting condition indicating a condition by which to delete the medical image stored in the medical image managing apparatus; and deleting the medical image stored in the medical image managing apparatus, based on the set deleting condition.

The method may further include adding an indicator, indicating whether to enable the medical image to be deleted, into the information about the medical image, and displaying the indicator.

The deleting condition may include a first deleting condition which is set based on a type of the first external storage device.

The deleting condition may include a second deleting condition which is set based on at least one of a residual capacity of a storage unit of the medical image managing apparatus, existence of a backup copy of the medical image, a time that elapses after the medical image is stored in the storage unit, and a frequency of use of the medical image.

The medical image managing method may further include: determining the first external storage device from among the plurality of external storage devices, based on the mapping information; and receiving the deleted medical image from the first external storage device according to a user inputting a recovery input to recover the deleted medical image.

The mapping information may further include information about an object corresponding to the deleted medical image, and the determining may include displaying the mapping information.

The method may further include receiving the recovery input for the deleted medical image according to the user inputting a selection input to select the displayed mapping information.

The method may further include displaying information indicating whether to enable the deleted medical image to be recovered.

The method may further include displaying information indicating whether the first external storage device is connected to the medical image managing apparatus as part of the information indicating whether to enable the deleted medical image to be recovered.

The mapping information may further include information about at least one of an examination kind of the object and a photographing part of the object.

The information about the first external storage device may include at least one of a type of the first external storage device and access information of the first external storage device, and the information about the medical image may include a unique identifier (UID) of the medical image.

According to one or more exemplary embodiments, there is provided a medical image managing apparatus including: a transmitter configured to transmit a backup copy of a medical image, which medical image is stored in the medical image managing apparatus, to a first external storage device which is selected from among a plurality of external storage devices; a storage configured to store mapping information comprising information about the medical image and information indicating the first external storage device from among a plurality of external storage devices to which the backup copy of the medical image is transmitted; and a controller configured to set a deleting condition by which the medical image stored in the medical image managing apparatus is deleted, and to delete the medical image stored in the medical image managing apparatus, based on the set deleting condition.

The medical image managing apparatus may be configured to add an indicator, indicating whether to enable the medical image to be deleted, into the information about the medical image, and to display the indicator.

The deleting condition may include a first deleting condition which is set based on a type of the first external storage device.

The deleting condition may include a second deleting condition which is set based on at least one of a residual capacity of a storage unit of the medical image managing apparatus, existence of a backup copy of a medical image, a time that elapses after the medical image is stored in the storage, and a frequency of use of the medical image.

The controller may be configured to determine the first external storage device from among the plurality of external storage devices based on the mapping information, and the medical image managing apparatus may further include a receiver configured to receive the deleted medical image from the first external storage device according to a user inputting a recovery input to recover the deleted medical image.

The mapping information may further include information about an object corresponding to the deleted medical image, and the medical image managing apparatus may further include a display configured to display the mapping information.

The receiver may be further configured to receive the recovery input for the deleted medical image according to the user inputting a selection input to select the displayed mapping information.

The display may be configured to display information indicating whether to enable the deleted medical image to be recovered.

The display may be configured to display the information indicating whether the first external storage device is connected to the medical image managing apparatus as part of the information indicating whether to enable the deleted medical image to be recovered.

The mapping information may further include information about at least one of an examination kind of the object and a photographing part of the object.

The information about the first external storage device may include at least one of a type of the first external storage device and access information of the first external storage device, and the information about the medical image may include a unique identifier (UID) of the medical image.

According to one or more exemplary embodiments, provided is a non-transitory computer-readable storage medium storing a computer program for executing the medical image managing method.

### BRIEF DESCRIPTION OF THE DRAWINGS

These and/or other aspects will become apparent and more readily appreciated from the following description of the exemplary embodiments, taken in conjunction with the accompanying drawings in which:
FIG. 1 is a diagram illustrating a medical image managing apparatus and a plurality of external storage devices according to an exemplary embodiment;
FIG. 2 is a flowchart of a medical image managing method according to an exemplary embodiment;
FIG. 3 is a flowchart of a medical image managing method according to another exemplary embodiment;
FIG. 4 is a diagram illustrating exemplary mapping information according to an exemplary embodiment;
FIG. 5 is a diagram for describing a method of setting a deleting condition of a medical image according to an exemplary embodiment;
FIG. 6 is a diagram illustrating an exemplary recovery list according to an exemplary embodiment; and
FIG. 7 is a diagram illustrating a configuration of a medical image managing apparatus according to an exemplary embodiment.

### DETAILED DESCRIPTION

Reference will now be made in detail to exemplary embodiments, examples of which are illustrated in the accompanying drawings, wherein like reference numerals refer to like elements throughout. In this regard, the exemplary embodiments may have different forms and should not be construed as being limited to the descriptions set forth herein. Accordingly, the exemplary embodiments are merely described below, by referring to the figures, to explain aspects of the present disclosure. Expressions such as "at least one of," when preceding a list of elements, modify the entire list of elements and do not modify the individual elements of the list.

The advantages, features and aspects of the exemplary embodiments will become apparent from the following description of the exemplary embodiments with reference to the accompanying drawings, which is set forth hereinafter. The exemplary embodiments may, however, be embodied in different forms and should not be construed as being limited to the exemplary embodiments set forth herein. Rather, these exemplary embodiments are provided so that this disclosure will be thorough and complete, and will fully convey the scope of the present disclosure to those of ordinary skill in the art.

Terms used herein will be briefly described, and the exemplary embodiments will be described in detail.

Terms used in the present disclosure have been selected as general terms which are widely used at present, in consideration of the functions of the exemplary embodiments, but may be altered according to the intent of an operator of ordinary skill in the art, conventional practice, or introduction of new technology. Also, if there is a term which is arbitrarily selected by the applicant in a specific case, a meaning of the term will be described in detail in a corresponding description portion of the exemplary embodiment. Therefore, the terms should be defined on the basis of the entire content of this specification instead of a simple name of each of the terms.

In the disclosure below, when the disclosure describes that a feature comprises (or includes or has) some elements, it should be understood that the feature may comprise (or include or has) only those elements, or the feature may comprise (or include or have) other elements as well as those elements, if there is no specific limitation. The term "module", as used herein, may refer to, but is not limited to, a software or hardware component, such as a Field Programmable Gate Array (FPGA) or an Application Specific Integrated Circuit (ASIC), which performs certain tasks. A module may advantageously be configured to reside in an addressable storage medium and be configured to execute on one or more processors. Thus, a module may include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. The functionality provided for in the components and modules may be combined into fewer components and modules or further separated into additional components and modules.

Exemplary embodiments capable of being easily implemented by those of ordinary skill in the art will now be described in detail with reference to the accompanying drawings. In the accompanying drawings, portions irrelevant to a description of the exemplary embodiments will be omitted for clarity.

According to an exemplary embodiment, the term "image" used herein may refer to multi-dimensional data composed of discrete image factors (for example, pixels in a two-dimensional (2D) image and pixels in a three-dimensional (3D) image). For example, an image may include a medical image of an object which is acquired by an X-ray apparatus, a computed tomography (CT) apparatus, a magnetic resonance imaging (MRI) apparatus, an ultrasonic apparatus, or another medical image photographing apparatus.

Moreover, according to an exemplary embodiment, the term "object" used herein may refer to a person, an animal, a part of the person, or a part of the animal. For example, an object may include an organ such as a liver, a heart, a womb, a brain, breasts, an abdomen, or the like, or a blood vessel. Also, the term "object" may refer to a phantom. According to an exemplary embodiment, the term phantom may refer to a material having a volume very close to a density of organisms and an effective atomic number, and may include a spherical phantom having a constitution similar to a human body.

Moreover, according to an exemplary embodiment, the term "user" as used herein may refer to a medical expert, and may be a doctor, a nurse, a medical technologist, a medical image expert, or the like, or may be an engineer repairing a medical apparatus. However, the term "user" is not limited thereto.

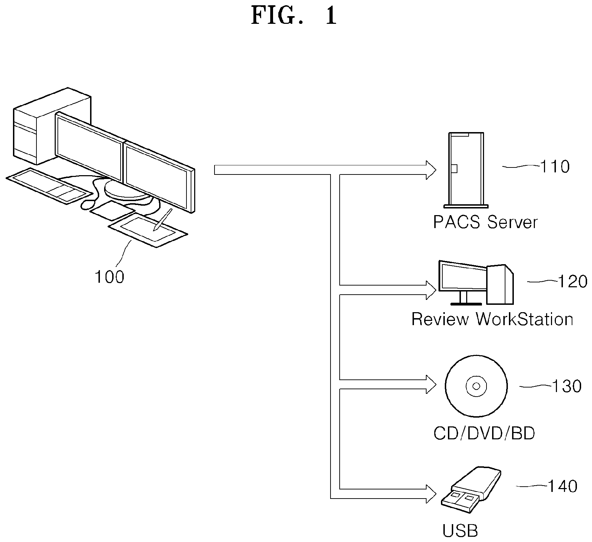

FIG. 1 is a diagram illustrating a medical image managing apparatus 100 and a plurality of external storage devices 110, 120, 130 and 140 according to an exemplary embodiment.

Referring to FIG. 1, the medical image managing apparatus 100 may be connected to the plurality of external storage devices 110, 120, 130 and 140. The medical image managing apparatus 100 according to an exemplary embodiment may include a central control device connected to a medical image photographing apparatus.

The medical image managing apparatus 100 may transmit a medical image stored therein to the plurality of external storage devices 110, 120, 130 and 140.

The plurality of external storage devices 110, 120, 130 and 140 may respectively include a picture archiving and communication system (PACS) server 110, a review workstation 120, a compact disk (CD)/digital versatile disk (DVD)/blue ray disk (BD) 130, and a universal serial bus (USB) 140, but are not limited thereto.

A storage space of the medical image managing apparatus 100 is limited, and thus, has a limited capacity to store all medical images that are continuously acquired. For this reason, a user may be required to store backup copies of the medical images in the plurality of external storage devices 110, 120, 130 and 140 by transmitting the medical images to the plurality of external storage devices 110, 120, 130 and 140.

When a medical image stored in the medical image managing apparatus 100 is transmitted to a first external storage device of the plurality of external storage devices 110, 120, 130 and 140, the medical image managing apparatus 100 may generate mapping information in which information about the first external storage device is mapped to information about the medical image.

Therefore, when a user desires to recover a medical image deleted from the medical image managing apparatus 100, the medical image managing apparatus 100 may easily recover the deleted medical image from the first external storage device on the basis of the mapping information.

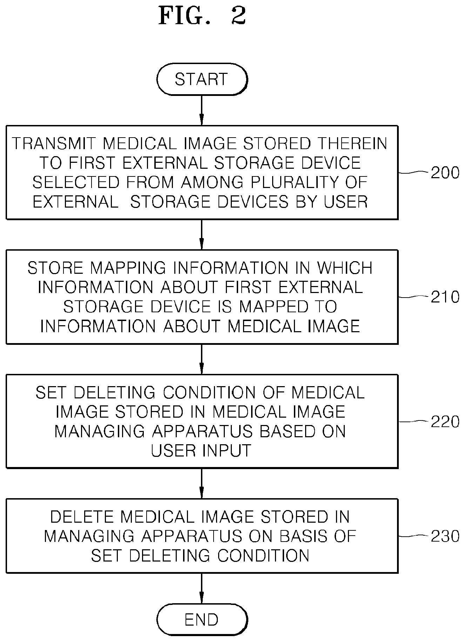

FIG. 2 is a flowchart of a medical image managing method according to an exemplary embodiment.

In operation 200, a medical image managing apparatus may transmit a medical image stored therein to a first external storage device selected from among a plurality of external storage devices by a user.

The medical image managing apparatus may select at least one or more medical images from among a plurality of medical images stored therein according to user input, and may transmit the selected medical image to the first external storage device.

According to an exemplary embodiment, the first external storage device may be an external storage device which is selected by the user from among the plurality of external storage devices connected to the medical image managing apparatus. It is understood, however, that the first external storage device is not required to be selected by the user, and may be automatically selected according to other exemplary embodiments.

In operation 210, the medical image managing apparatus may store mapping information in which information about the first external storage device is mapped to information about the medical image.

The information about the medical image may be used by the user to identify and classify a type of the transmitted medical image.

The information about the first external storage device is information for easily identifying an external storage device to which the medical image is transmitted, by identifying a type of the external storage device and a transmitted date.

The mapping information is information which is stored and which maps the medical image selected by the user with the first external storage device receiving the medical image so as to easily determine which of the plurality of external storage devices stores the medical image.

In operation 220, a deletion condition of a medical image stored in the medical image managing apparatus may be set based on user input.

The medical image managing apparatus may delete at least one of a plurality of medical images transmitted from the medical image managing apparatus to the first external storage device. The medical image managing apparatus may delete the at least one medical image based on various types of deletion conditions according to the type of the first external storage device.

According to an exemplary embodiment, the deletion condition may be set based on at least one of a residual capacity of a storage unit, whether there is a backup copy of a medical image, a time that elapses after the medical image is stored in the storage unit, and a frequency of use of the medical image. For example, according to an exemplary embodiment, the deleting condition may include a deletion condition to delete the medical image when the residual capacity of the storage unit is 10% or less, a deleting condition to delete the medical image when there is a backup copy of a medical image, a deleting condition to delete the medical image when three months elapses after the medical image is stored in the storage unit, and a deleting condition to delete the medical image when the number of times the user uses the medical image for treating a patient or for uses the medical image for other purpose is two times or less.

In operation 230, the medical image managing apparatus may delete the medical image on the basis of the set deleting condition.

The medical image managing apparatus may delete a medical image, corresponding to the set deleting condition, from among all of the medical images stored therein. Also, the medical image managing apparatus may delete a medical image, corresponding to the set deleting condition, among arbitrary medical images which are selected from among the stored medical images by the user.

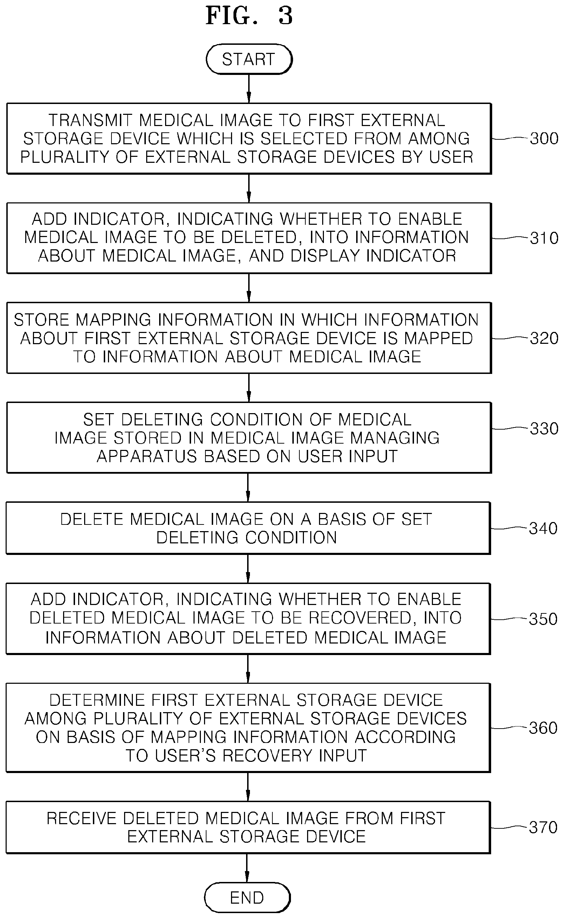

FIG. 3 is a flowchart of a medical image managing method according to another exemplary embodiment.

In operation 300, a medical image managing apparatus may transmit a medical image to a first external storage device which is selected from among a plurality of external storage devices.

In operation 310, the medical image managing apparatus may add an indicator, indicating whether to enable the medical image to be deleted, into information about the medical image, and display the indicator.

Although the medical image transmitted to the first external storage device may be deleted from the medical image managing apparatus, the deleted medical image may be recovered by restoring a backup copy of the medical image. However, when a medical image which is not transmitted to the first external storage device is deleted, the deleted medical image cannot be recovered.

In order to overcome such a limitation, the medical image managing apparatus according to an exemplary embodiment may add the indicator, indicating whether to enable the medical image to be deleted, into the information about the medical image so as to enable only medical images which are transmitted to the first external storage device to be deleted later.

In operation 320, the medical image managing apparatus may store mapping information in which information about the first external storage device is mapped to the information about the medical image.

In operation 330, a deleting condition of a medical image stored in the medical image managing apparatus may be set based on user input.

According to an exemplary embodiment, examples of the deleting condition may include a deleting condition that deletes medical images in a descending order of acquisition timings of the medical images, a deleting condition that deletes medical images in a first-in, first-out order from the first-acquired medical image (a first-in, first-out (FIFO) deleting condition), and a deleting condition that deletes a least recently used medical image (a least recently used (LRU) deleting condition).

The deleting condition may be set based on various criteria, including for example, at least one of a residual capacity of a storage unit, whether there is a backup copy of a medical image, a time that elapses after the medical image is stored in the storage unit, and a frequency of use of the medical image.

In operation 340, the medical image managing apparatus may delete a medical image on the basis of the set deleting condition.

When user inputs instructions to delete a medical image after the deleting condition is set, the medical image managing apparatus may delete medical images stored therein on the basis of the deleting condition.

In operation 350, the medical image managing apparatus may add an indicator, indicating whether to enable the deleted medical image to be recovered, into information about the deleted medical image.

When a backup copy of a medical image to be used to recover the medical image is stored in the first external storage device and the medical image managing apparatus is connected to the first external storage device, the medical image managing apparatus may recover the deleted medical image.

When the medical image managing apparatus is not connected to the first external storage device, the medical image managing apparatus cannot receive the medical image stored in the first external storage device, and thus, the deleted medical image cannot be recovered. Also, when a backup copy of a medical image to be used to recover the medical image is not stored in the first external storage device (for example, when a backup file is deleted from the first external storage device, or when the medical image is not transmitted to the first external storage device), the medical image to be transmitted to the medical image managing apparatus is not stored in the first external storage device, and thus, the medical image managing apparatus cannot recover the deleted medical image.

In operation 360, the medical image managing apparatus may determine the first external storage device among the plurality of external storage devices on the basis of the mapping information according to the user's recovery input.

The medical image managing apparatus may determine the first external storage device among the plurality of external storage devices on the basis of the mapping information according to the user's recovery input for the deleted medical image. The user may obtain information of each medical image and information, mapped to the information of each medical image, of the first external storage device by using the mapping information which is stored in operation 320.

When the user selects a medical image to be recovered on the basis of the mapping information, the user may determine the first external storage device recorded in the mapping information.

In operation 370, the medical image managing apparatus may receive the deleted medical image from the first external storage device.

The medical image managing apparatus may receive a selected medical image from the first external storage device according to the user's recovery input.

The first external storage device is a storage device which stores the medical image, and the user may determine each of the first external storage devices respectively mapped to each medical image on the basis of information recorded in the mapping information.

When the user selects a medical image and inputs a recovery input, the medical image managing apparatus may receive the medical image from the first external storage device.

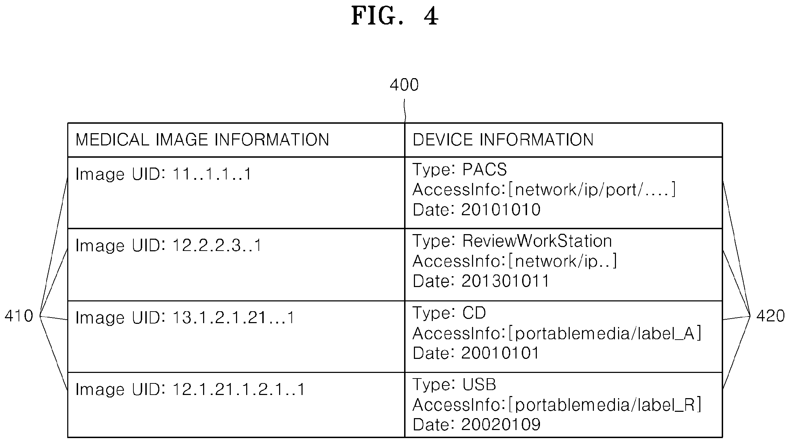

FIG. 4 is a diagram illustrating medical image information and device information according to an exemplary embodiment.

Referring to FIG. 4, according to an exemplary embodiment, a medical image is transmitted from a medical image managing apparatus to a first external storage device, and then the medical image managing apparatus stores an image information table 400 that shows information 410 about each medical image and information 420 about the first external storage device receiving the medical image.

The information 410 about each medical image may include a unique image identifier (UID), access information (Access Info) about the first external storage device, and a date at which the medical image was transmitted from the medical image managing apparatus to the first external storage device.

The image UID is an identifier (ID) for identifying medical images.

The access information (Access Info) about the first external storage device is used when a connection between the medical image managing apparatus and the first external storage device is needed (for example, when recovering the medical image). The access information (Access Info) about the first external storage device may selectively include content to record according to the user's preferences. For example, the access information (Access Info) about the first external storage device may include a network Internet protocol (IP) address (e.g., when the first external storage device is connected to the medical image managing apparatus over the Internet), a port number, an application entity title, external hard disk connection information, and a CD label.

An indicator, which enables a medical image (e.g., a medical image transmitted from the medical image managing apparatus to the first external storage device) to be deleted later, may be added into information about the medical image.

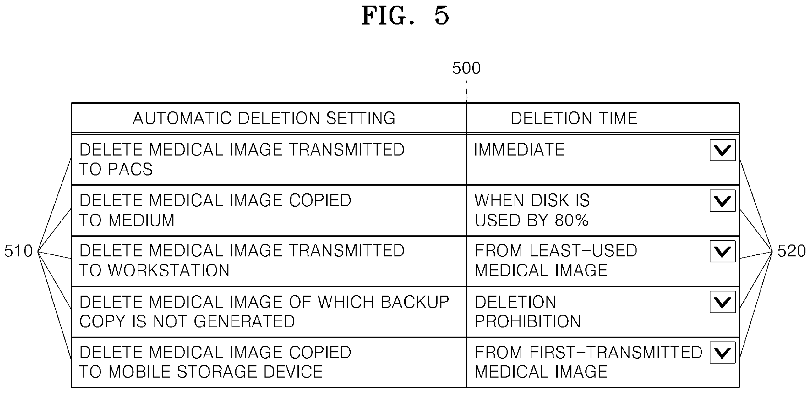

FIG. 5 is a diagram for describing a method of setting a deleting condition of a medical image according to an exemplary embodiment.

Referring to FIG. 5, a user may input a deletion command (for example, a cleanup command) for arranging a storage space of a medical image managing apparatus, and then may set a deleting condition in a deleting condition setting table 500 in which a deleting condition of a medical image is set by the medical image managing apparatus.

According to an exemplary embodiment, a medical image, which does not have an indicator to enable the medical image to be deleted, among pieces of information about medical images stored in the medical image managing apparatus, may not be deleted even when the deletion command is input.

The deleting condition setting table 500 may include an automatic deletion setting 510, which sets types of images to be automatically deleted, and a deletion time 520 that indicates a deleting condition.

According to an exemplary embodiment, the automatic deletion setting 510 and the deletion time 520 may show that different deleting conditions are set according to devices which are used. For example, a setting may be made in which a medical image transmitted to a PACS server may be immediately deleted, a medical image copied to a recording medium may be deleted when at least 80% of a corresponding disk is used, a medical image transmitted to a workstation may be deleted when the medical image is least used among a plurality of medical images, a medical image of which a backup copy is not generated may be prohibited from being deleted, and a medical image copied to a mobile storage device may be deleted in an order starting with a first-transmitted medical image.

In addition, a deleting condition may be set according to the user's preferences, and for example, various settings may be set based on a type of the first external storage device. As examples of the various settings, medical images may be deleted in an order starting with the most recently captured medical image, the medical images may be deleted at random, the medical images may be deleted when a predetermined time elapses after transmission, the medical images may be deleted when at least 70% of a disk is used, the medical images may be deleted in an order starting with the most-used medical image, and the medical images may be deleted in an order starting with a first-transmitted medical image among a plurality of medical images.

When a setting of the deleting condition is ended, the user may start to delete medical images stored in the medical image managing apparatus by using a delete button, an execute short key, or any other input technique known to those skilled in the art.

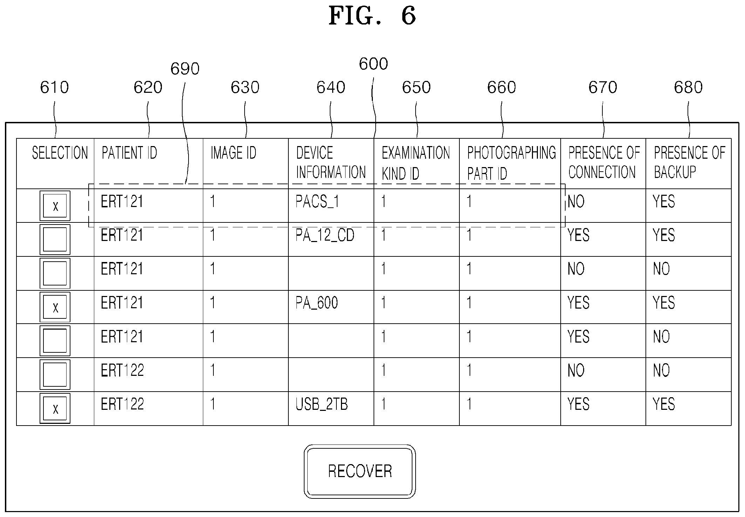

FIG. 6 is a diagram for describing information about backup of medical images according to an exemplary embodiment.

Referring to FIG. 6, a medical image backup information list 600 indicating whether to enable a deleted medical image to be recovered may include a selection presence item 610 indicating whether a medical image for a backup is selected, a patient ID item 620, an image ID item 630, a device information item 640 indicating information of the first external storage device storing a backup copy, an examination kind ID (exam ID) item 650, a photographing part ID (series ID) item 660, a connection presence item 670 indicating whether to enable the medical image managing apparatus to be connected to the first external storage device, a backup presence item 680 indicating whether a medical image has been transmitted from the medical image managing apparatus to the first external storage device, and mapping information 690.

When a backup copy is not generated by transmitting a medical image from the medical image managing apparatus to the first external storage device, a user will be unable to recover the medical image, and thus, a user may check the presence of a backup through the backup presence item 680 of the medical image backup information list 600.

The user may determine, through the connection presence item 670, whether to enable the medical image managing apparatus to be connected to the first external storage device. The user may determine whether to enable the medical image managing apparatus to be connected to the first external storage device, according to whether the medical image managing apparatus is connected to a portable storage device, such as the USB 140 or the CD/DVD/BD 130, and whether the medical image managing apparatus is connected to a server.

When the connection presence item 670 indicates "YES", the medical image managing apparatus is connected to the first external storage device, and thus, the medical image managing apparatus may receive a medical image from the first external storage device.

When the medical image managing apparatus is not connected to the first external storage device, a user is unable to recover a medical image to be recovered from the first external storage device, until the medical image managing apparatus becomes connected to the first external storage device.

The user may obtain information of the first external storage device storing a medical image by using the device information item 640. When the connection presence item 670 indicates that the medical image managing apparatus is not connected to the first external storage device, the medical image managing apparatus may be connected to the first external storage device indicated by the device information item 640. At this time, the connection presence item 670 of the medical image backup information list 600 may be changed from "NO" to "YES", thereby indicating that the first external storage device storing the medical image is connected to the medical image managing apparatus.

For example, when the backup presence item 680 indicates "YES", the connection presence item 670 indicates "NO", and the device information item 640 indicates information "PACS_1", the first external storage device "PACS_1" may then be connected to the medical image managing apparatus so as to enable recovery of a backup medical image. At this time, the connection presence item 670 is changed to "YES".

After selection of a medical image to be recovered is made, the user may press a recover button to start to recover the selected medical image.

The mapping information 690 may include patient ID item 620 indicating a patient ID, an image ID item 630 indicating an image ID, a device information item 640 indicating information related to the first external storage device storing a backup copy, an examination kind ID item 650 indicating a kind of examination, and a photographing part ID item 660 indicating a photographing part ID.

The mapping information 690 may further include information about an object corresponding to a deleted medical image.

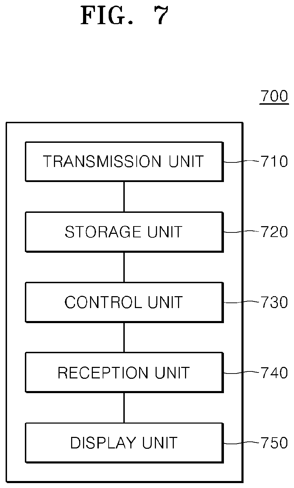

FIG. 7 is a diagram illustrating a configuration of a medical image managing apparatus 700 according to an exemplary embodiment.

Referring to FIG. 7, the medical image managing apparatus 700 may include a transmission unit 710 (e.g., transmitter), a storage unit 720 (e.g., storage or memory), a control unit 730 (e.g., controller or processor), a reception unit 740 (e.g., receiver), and a display unit 750 (e.g., display).

According to an exemplary embodiment, the medical image managing apparatus 700 of FIG. 7 may perform the same function as the functions performed by the above-described medical image managing apparatus 100 of FIG. 1.

The transmission unit 710 may transmit a medical image, stored in the medical image managing apparatus 700, to a first external storage device which is selected from among a plurality of external storage devices by a user.

The medical image managing apparatus 700 may be connected to the first external storage device in a wired manner, a wireless manner, or a combination thereof. The medical image may be transmitted from the medical image managing apparatus 700 to the first external storage device which is selected from among the plurality of external storage devices by the user.

The medical image may be acquired by using an MRI apparatus, a CT apparatus, a position emission tomography (PET) apparatus, or an X-ray apparatus. The medical image managing apparatus 700 may include a workstation that acquires the medical image and temporarily stores the acquired medical image, but is not limited thereto.

The medical image transmitted to the first external storage device may include an image based on the digital imaging and communications in medicine (DICOM) standard.

The storage unit 720 may store information about the first external storage device and information about the medical image which may be the same as the information described above with reference to FIG. 5.

The storage unit 720 may further store mapping information in which the medical image, the information about the first external storage device, and the information about the medical image are mapped to each other.

The storage unit 720 may store the medical image which is acquired by photographing an object by using a medical apparatus such as an MRI apparatus, a CT apparatus, a PET apparatus, or an X-ray apparatus.

The storage unit 720 may store a UID as information for identifying the medical image, or store information about the medical apparatus acquiring the medical image or information about an object which is the subject of the medical image which has been captured.

Moreover, the storage unit 720 may store information of the first external storage device and a date at which the medical image was transmitted as the information about the first external storage device receiving the medical image. The storage unit 720 may store the mapping information in which the information about the medical image is mapped to the information about the first external storage device storing the medical image.

The control unit 730 may set a deleting condition of the medical image stored in the medical image managing apparatus 700 on the basis of user input, and delete the medical image on the basis of the set deleting condition.

The control unit 730 may delete the medical image and may determine the first external storage device from among the plurality of external storage devices on the basis of the mapping information according to the user's recovery input for the deleted medical image.

The control unit 730 may receive a deletion input command from the user to delete the medical image stored in the medical image managing apparatus 700, and may enable the reception unit 740 to receive the medical image from the first external storage device according to the recovery input.

The reception unit 740 may receive a backup copy of the deleted medical image from the first external storage device.

When a user desires to recover the medical image deleted from the medical image managing apparatus 700, the reception unit 740 may receive the deleted medical image from the first external storage device storing the medical image.

The reception unit 740 may receive the medical image from the first external storage device in a wired manner, a wireless manner, or a combination thereof.

The display unit 750 may display an indicator which indicates whether to enable the medical image to be deleted, and the indicator may be included in the information about the medical image.

When user input for deleting the medical image stored in the medical image managing apparatus 700 is input, the display unit 750 may display a table in which a deleting condition of the medical image is set. Also, when a user input for recovering the medical image deleted from the medical image managing apparatus 700 is input, the display unit 750 may display the backup information list 600 for the medical image.

The exemplary embodiments may be written as computer programs and may be implemented in general-use digital computers that execute the programs using a computer readable recording medium.

Examples of the computer readable recording media include magnetic storage media (e.g., ROM, floppy disks, hard disks, etc.) and optical recording media (e.g., CD-ROMs, or DVDs).

It should be understood that the exemplary embodiments described herein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described above with reference to the figures, it will be understood by those of ordinary skill in the art that various changes in form and details may be made therein without departing from the scope of the present disclosure as defined by the following claims.

## Claims

1. A medical image managing method to be performed using a medical image managing apparatus, the medical image managing method comprising:
transmitting a backup copy of a medical image, which medical image is stored in the medical image managing apparatus, to a first external storage device which is selected from among a plurality of external storage devices;
storing mapping information, comprising information about the medical image and information indicating the first external storage device from among a plurality of external storage devices, to which the backup copy of the medical image is transmitted;
setting a deleting condition indicating a condition by which to delete the medical image stored in the medical image managing apparatus; and
deleting the medical image stored in the medical image managing apparatus, based on the set deleting condition.

2. The medical image managing method of claim 1, further comprising retrieving a backup copy of the medical image from the first external storage device from among plurality of external storage devices, to which the backup copy of the medical image was transmitted, based on the mapping information.

3. The medical image managing method of claim 1 or 2, further comprising adding an indicator, indicating whether the medical image may be deleted, into the information about the medical image, and displaying the indicator.

4. The medical image managing method of claim 1, wherein the deleting condition comprises a first deleting condition which is set based on a type of the first external storage device, and preferably the deleting condition comprises a second deleting condition which is set based on at least one of a residual capacity of a storage unit of the medical image managing apparatus, existence of a backup copy of the medical image, a time that elapses after the medical image is stored in the storage unit, and a frequency of use of the medical image.

5. The medical image managing method of claim 1, further comprising:
determining the first external storage device from among the plurality of external storage devices based on the mapping information; and
receiving the deleted medical image from the first external storage device according to a user inputting a recovery input to recover the deleted medical image.

6. The medical image managing method of claim 5, wherein,
the mapping information further comprises information about an object corresponding to the deleted medical image, wherein the medical image is acquired of the object by photographing the object by using a medical apparatus such as an MRI apparatus, a CT apparatus, a PET apparatus, or an X-ray apparatus, and
the determining comprises displaying the mapping information.

7. The medical image managing method of claim 6, further comprising receiving the recovery input for the deleted medical image according to the user inputting a selection, which is input to select the displayed mapping information.

8. A medical image managing apparatus comprising:
a transmitter configured to transmit a backup copy of a medical image, which medical image is stored in the medical image managing apparatus, to a first external storage device which is selected from among a plurality of external storage devices;
a storage configured to store mapping information, comprising information about the medical image and information indicating the first external storage device from among plurality of external storage devices, to which the backup copy of the medical image is transmitted; and
a controller configured to set a deleting condition by which the medical image stored in the medical image managing apparatus is deleted, to delete the medical image stored in the medical image managing apparatus, based on the set deleting condition.

9. The medical image managing apparatus of claim 8, wherein the controller is additionally configured to retrieve a backup copy of the medical image from the first external storage device from among plurality of external storage devices, to which the backup copy of the medical image was transmitted, based on the mapping information.

10. The medical image managing apparatus of claim 8 or 9, wherein the medical image managing apparatus is configured to add an indicator, indicating whether the medical image may be deleted, into the information about the medical image, and to display the indicator.

11. The medical image managing apparatus of claim 8, wherein the deleting condition comprises a first deleting condition which is set based on a type of the first external storage device, wherein the deleting condition preferably comprises a second deleting condition which is set based on at least one of a residual capacity of a storage unit of the medical image managing apparatus, existence of a backup copy of a medical image, a time that elapses after the medical image is stored in the storage, and a frequency of use of the medical image.

12. The medical image managing apparatus of claim 8, wherein,
the controller is configured to determine the first external storage device from among the plurality of external storage devices based on the mapping information; and
the medical image managing apparatus further comprises a receiver configured to receive the deleted medical image from the first external storage device according to a user inputting a recovery input to recover the deleted medical image.

13. The medical image managing apparatus of claim 12, wherein,
the mapping information further comprises information about an object corresponding to the deleted medical image, wherein the medical image is acquired of the object by photographing the object by using a medical apparatus such as an MRI apparatus, a CT apparatus, a PET apparatus, or an X-ray apparatus, and
the medical image managing apparatus further comprises a display configured to display the mapping information.

14. The medical image managing apparatus of claim 13, wherein the receiver is configured to receive the recovery input for the deleted medical image according to the user inputting a selection, which is input to select displayed mapping information.

15. A non-transitory computer-readable storage medium storing a computer program for performing the steps of the medical image managing method of claim 1 when executed on a computer.

## Patentansprüche

1. Verfahren zur Verwaltung medizinischer Bilder, wobei das Verfahren zur Verwaltung medizinischer Bilder unter Verwendung einer medizinischen Bildgebungsvorrichtung durchgeführt wird und Folgendes umfasst:
Übertragen einer Sicherungskopie eines in der Vorrichtung zur Verwaltung medizinischer Bilder gespeicherten medizinischen Bildes an eine erste externe Speichervorrichtung, die aus einer Mehrzahl von externen Speichervorrichtungen ausgewählt wird;
Speichern von Abbildungsinformationen umfassend Informationen über das medizinische Bild und Informationen, die die erste externe Speichervorrichtung aus einer Mehrzahl von externen Speichervorrichtungen, an die die Sicherungskopie des medizinischen Bildes übertragen wird, anzeigen;
Einstellen einer Löschungsbedingung, die eine Bedingung anzeigt, unter der das in der Vorrichtung zur Verwaltung medizinischer Bilder gespeicherte medizinische Bild gelöscht wird; und
Löschen des in der Vorrichtung zur Verwaltung medizinischer Bilder gespeicherten medizinischen Bildes basierend auf der eingestellten Löschungsbedingung.

2. Verfahren zur Verwaltung medizinischer Bilder nach Anspruch 1, weiterhin umfassend das Abrufen einer Sicherungskopie des medizinischen Bildes aus der ersten externen Speichervorrichtung aus der Mehrzahl von externen Speichervorrichtungen, an die die Sicherungskopie des medizinischen Bildes gesendet wurde, basierend auf den Abbildungsinformationen.

3. Verfahren zur Verwaltung medizinischer Bilder nach Anspruch 1 oder 2, weiterhin umfassend das Hinzufügen eines Anzeigers, der anzeigt, ob das medizinische Bild gelöscht werden kann, zu den Informationen über das medizinische Bild, und Anzeigen des Anzeigers.

4. Verfahren zur Verwaltung medizinischer Bilder nach Anspruch 1, wobei die Löschungsbedingung eine erste Löschungsbedingung umfasst, die basierend auf der Art der ersten externen Speichervorrichtung eingestellt wird, und wobei die Löschungsbedingung vorzugsweise eine zweite Löschungsbedingung umfasst, die basierend auf wenigstens einem der Folgenden eingestellt wird: einer Restkapazität einer Speichereinheit der Vorrichtung zur Verwaltung medizinischer Bilder, der Existenz einer Sicherungskopie des medizinischen Bildes, einer Zeit, die nach der Speicherung des medizinischen Bildes in der Speichereinheit abgelaufen ist, und einer Häufigkeit der Verwendung des medizinischen Bildes.

5. Verfahren zur Verwaltung medizinischer Bilder nach Anspruch 1, das weiterhin Folgendes umfasst:
Bestimmen der ersten externen Speichervorrichtung aus der Mehrzahl von Speichervorrichtungen basierend auf den Abbildungsinformationen; und
Empfangen des gelöschten medizinischen Bildes aus der ersten externen Speichervorrichtung gemäß einer von einem Benutzer eingegebenen Wiederherstellungseingabe zur Wiederherstellung des gelöschten medizinischen Bildes.

6. Verfahren zur Verwaltung medizinischer Bilder nach Anspruch 5, wobei
die Abbildungsinformationen weiterhin Informationen über ein dem gelöschten medizinischen Bild entsprechendes Objekt umfassen, wobei das medizinische Bild des Objekts durch eine Bildgebung des Objekts unter Verwendung einer medizinischen Vorrichtung wie zum Beispiel einer MRT-Vorrichtung, einer CT-Vorrichtung, einer PET-Vorrichtung oder einer Röntgenvorrichtung erhalten wird, und
das Bestimmen das Anzeigen der Abbildungsinformationen umfasst.

7. Verfahren zur Verwaltung medizinischer Bilder nach Anspruch 6, weiterhin umfassend den Empfang der Wiederherstellungseingabe für das gelöschte medizinische Bild gemäß der von einem Benutzer eingegebenen Auswahl, die eingegeben wird, um die angezeigten Abbildungsinformationen auszuwählen.

8. Vorrichtung zur Verwaltung medizinischer Bilder, die Folgendes umfasst:
eine Übertragungseinrichtung, die konfiguriert ist zum Übertragen einer Sicherungskopie eines in der Vorrichtung zur Verwaltung medizinischer Bilder gespeicherten medizinischen Bildes an eine erste externe Speichervorrichtung, die aus einer Mehrzahl von externen Speichervorrichtungen ausgewählt wird;
einen Speicher, der konfiguriert ist zum Speichern von Abbildungsinformationen umfassend Informationen über das medizinische Bild und Informationen, die die erste externe Speichervorrichtung aus der Mehrzahl von externen Speichervorrichtungen, an die die Sicherungskopie des medizinischen Bildes übertragen wird, anzeigen; und
eine Steuerung, die konfiguriert ist zum Einstellen einer Löschungsbedingung, unter der das in der Vorrichtung zur Verwaltung medizinischer Bilder gespeicherte medizinische Bild gelöscht wird, und zum Löschen des in der Vorrichtung zur Verwaltung medizinischer Bilder gespeicherten medizinischen Bildes basierend auf der eingestellten Löschungsbedingung.

9. Vorrichtung zur Verwaltung medizinischer Bilder nach Anspruch 8, wobei die Steuerung zusätzlich konfiguriert ist zum Abrufen einer Sicherungskopie des medizinischen Bildes aus der ersten externen Speichervorrichtung aus der Mehrzahl von externen Speichervorrichtungen, an die die Sicherungskopie des medizinischen Bildes gesendet wurde, basierend auf den Abbildungsinformationen.

10. Vorrichtung zur Verwaltung medizinischer Bilder nach Anspruch 8 oder 9, wobei die Vorrichtung zur Verwaltung medizinischer Bilder konfiguriert ist zum Hinzufügen eines Anzeigers, der anzeigt, ob das medizinische Bild gelöscht werden kann, zu den Informationen über das medizinische Bild, und zum Anzeigen des Anzeigers.

11. Vorrichtung zur Verwaltung medizinischer Bilder nach Anspruch 8, wobei die Löschungsbedingung eine erste Löschungsbedingung umfasst, die basierend auf der Art der ersten externen Speichervorrichtung eingestellt wird, wobei die Löschungsbedingung vorzugsweise eine zweite Löschungsbedingung umfasst, die basierend auf wenigstens einem der Folgenden eingestellt wird: einer Restkapazität einer Speichereinheit der Vorrichtung zur Verwaltung medizinischer Bilder, der Existenz einer Sicherungskopie des medizinischen Bildes, einer Zeit, die nach der Speicherung des medizinischen Bildes in der Speichereinheit abgelaufen ist, und einer Häufigkeit der Verwendung des medizinischen Bildes.

12. Vorrichtung zur Verwaltung medizinischer Bilder nach Anspruch 8, wobei
die Steuerung konfiguriert ist zum Bestimmen der ersten externen Speichervorrichtung aus der Mehrzahl von Speichervorrichtungen basierend auf den Abbildungsinformationen; und
die Vorrichtung zur Verwaltung medizinischer Bilder weiterhin einen Empfänger umfasst, der konfiguriert ist zum Empfangen des gelöschten medizinischen Bildes aus der ersten externen Speichervorrichtung gemäß einer von einem Benutzer eingegebenen Wiederherstellungseingabe zur Wiederherstellung des gelöschten medizinischen Bildes.

13. Vorrichtung zur Verwaltung medizinischer Bilder nach Anspruch 12, wobei
die Abbildungsinformationen weiterhin Informationen über ein dem gelöschten medizinischen Bild entsprechendes Objekt umfassen, wobei das medizinische Bild des Objekts durch eine Bildgebung des Objekts unter Verwendung einer medizinischen Vorrichtung wie zum Beispiel einer MRT-Vorrichtung, einer CT-Vorrichtung, einer PET-Vorrichtung oder einer Röntgenvorrichtung erhalten wird, und
die Vorrichtung zur Verwaltung medizinischer Bilder weiterhin eine Anzeige umfasst, die zum Anzeigen der Abbildungsinformationen konfiguriert ist.

14. Vorrichtung zur Verwaltung medizinischer Bilder nach Anspruch 13, wobei der Empfänger konfiguriert ist zum Empfangen der Wiederherstellungseingabe für das gelöschte medizinische Bild gemäß der von einem Benutzer eingegebenen Auswahl, die eingegeben wird, um die angezeigten Abbildungsinformationen auszuwählen.

15. Nicht flüchtiges computerlesbares Speichermedium, auf dem ein Computerprogramm gespeichert ist zur Durchführung der Schritte des Verfahrens zur Verwaltung medizinischer Bilder nach Anspruch 1, wenn es auf einem Computer ausgeführt wird.

## Revendications

1. Procédé de gestion d'image médicale destiné à être réalisé au moyen d'un dispositif de gestion d'image médicale, le procédé de gestion d'image médicale comprenant :
la transmission d'une copie de sauvegarde d'une image médicale, laquelle image médicale est enregistrée dans le dispositif de gestion d'image médicale, auprès d'un premier dispositif d'enregistrement externe choisi parmi une pluralité de dispositifs d'enregistrement externes,
l'enregistrement d'informations de correspondance, comprenant des informations concernant l'image médicale et des informations indicatrices du premier dispositif d'enregistrement externe, parmi une pluralité de dispositifs d'enregistrement externes, auprès duquel la copie de sauvegarde de l'image médicale a été transmise,
l'établissement d'une condition de suppression indicatrice d'une condition selon laquelle l'image médicale enregistrée dans le dispositif de gestion d'image médicale doit être supprimée, et
la suppression de l'image médicale enregistrée dans le dispositif de gestion d'image médicale, compte tenu de la condition de suppression établie.

2. Procédé de gestion d'image médicale selon la revendication 1, comprenant en outre la récupération d'une copie de sauvegarde de l'image médicale auprès du premier dispositif d'enregistrement externe, parmi pluralité de dispositifs d'enregistrement externes, auquel la copie de sauvegarde de l'image médicale avait été transmise, compte tenu des informations de correspondance.

3. Procédé de gestion d'image médicale selon la revendication 1 ou 2, comprenant en outre l'ajout, aux informations concernant l'image médicale, d'un indicateur indiquant si l'image médicale peut être supprimée, et l'affichage de l'indicateur.

4. Procédé de gestion d'image médicale selon la revendication 1, dans lequel la condition de suppression comprend une première condition de suppression qui est établie compte tenu du type du premier dispositif d'enregistrement externe, et la condition de suppression comprend de préférence une deuxième condition de suppression qui est établie compte tenu de la capacité résiduelle d'une unité d'enregistrement du dispositif de gestion d'image médicale, de l'existence d'une copie de sauvegarde de l'image médicale, du temps qui s'est écoulé depuis que l'image médicale a été enregistrée dans l'unité d'enregistrement, et/ou de la fréquence d'utilisation de l'image médicale.

5. Procédé de gestion d'image médicale selon la revendication 1, comprenant en outre :
la détermination du premier dispositif d'enregistrement externe parmi la pluralité de dispositifs d'enregistrement externes compte tenu des informations de correspondance, et
la réception de l'image médicale supprimée, en provenance du premier dispositif d'enregistrement externe, conformément à la saisie d'une entrée de restauration visant à la restauration de l'image médicale supprimée.

6. Procédé de gestion d'image médicale selon la revendication 5, dans lequel
les informations de correspondance comprennent en outre des informations concernant un objet correspondant à l'image médicale supprimée, ladite image médicale de l'objet étant prise par photographie de l'objet au moyen d'un appareil médical tel qu'un appareil à IRM, un tomodensitomètre, une caméra à positons ou un appareil de radiographie, et
la détermination comprend l'affichage des informations de correspondance.

7. Procédé de gestion d'image médicale selon la revendication 6, comprenant en outre la réception de l'entrée de restauration concernant l'image médicale supprimée conformément à une entrée, par l'utilisateur, d'une sélection, qui est réalisée pour sélectionner les informations de correspondance affichées.

8. Dispositif de gestion d'image médicale comprenant :
un transmetteur conçu pour transmettre une copie de sauvegarde d'une image médicale, laquelle image médicale est enregistrée dans le dispositif de gestion d'image médicale, auprès d'un premier dispositif d'enregistrement externe choisi parmi une pluralité de dispositifs d'enregistrement externes,
un enregistreur conçu pour enregistrer des informations de correspondance, comprenant des informations concernant l'image médicale et des informations indicatrices du premier dispositif d'enregistrement externe, parmi la pluralité de dispositifs d'enregistrement externes, auquel la copie de sauvegarde de l'image médicale a été transmise, et
un organe de commande conçu pour établir une condition de suppression selon laquelle l'image médicale enregistrée dans le dispositif de gestion d'image médicale est supprimée, et pour supprimer l'image médicale enregistrée dans le dispositif de gestion d'image médicale, compte tenu de la condition de suppression établie.

9. Dispositif de gestion d'image médicale selon la revendication 8, dans lequel l'organe de commande est conçu en outre pour récupérer une copie de sauvegarde de l'image médicale auprès du premier dispositif d'enregistrement externe, parmi la pluralité de dispositifs d'enregistrement externes, auquel la copie de sauvegarde de l'image médicale avait été transmise, compte tenu des informations de correspondance.

10. Dispositif de gestion d'image médicale selon la revendication 8 ou 9, dans lequel le dispositif de gestion d'image médicale est conçu pour ajouter, aux informations concernant l'image médicale, un indicateur indiquant si l'image médicale peut être supprimée, et pour afficher l'indicateur.

11. Dispositif de gestion d'image médicale selon la revendication 8, dans lequel la condition de suppression comprend une première condition de suppression qui est établie compte tenu du type du premier dispositif d'enregistrement externe, ladite condition de suppression comprenant de préférence une deuxième condition de suppression qui est établie compte tenu de la capacité résiduelle d'une unité d'enregistrement du dispositif de gestion d'image médicale, de l'existence d'une copie de sauvegarde d'une image médicale, du temps qui s'est écoulé depuis que l'image médicale a été enregistrée dans l'unité d'enregistrement, et/ou de la fréquence d'utilisation de l'image médicale.

12. Dispositif de gestion d'image médicale selon la revendication 8, dans lequel
l'organe de commande est conçu pour déterminer le premier dispositif d'enregistrement externe parmi la pluralité de dispositifs d'enregistrement externes compte tenu des informations de correspondance, et
le dispositif de gestion d'image médicale comprend en outre un récepteur conçu pour recevoir l'image médicale supprimée, en provenance du premier dispositif d'enregistrement externe, conformément à la saisie d'une entrée de restauration visant à la restauration de l'image médicale supprimée.

13. Dispositif de gestion d'image médicale selon la revendication 12, dans lequel
les informations de correspondance comprennent en outre des informations concernant un objet correspondant à l'image médicale supprimée, ladite image médicale de l'objet étant prise par photographie de l'objet au moyen d'un appareil médical tel qu'un appareil à IRM, un tomodensitomètre, une caméra à positons, ou un appareil de radiographie, et
le dispositif de gestion d'image médicale comprend en outre un écran conçu pour afficher les informations de correspondance.

14. Dispositif de gestion d'image médicale selon la revendication 13, dans lequel le récepteur est conçu pour recevoir l'entrée de restauration concernant l'image médicale supprimée conformément à une entrée, par l'utilisateur, d'une sélection, qui est réalisée pour sélectionner les informations de correspondance affichées.

15. Support d'enregistrement non transitoire lisible par ordinateur, sur lequel est enregistré un programme informatique permettant de réaliser les étapes du procédé de gestion d'image médicale selon la revendication 1 lorsqu'il est exécuté sur un ordinateur.
